# EUROPÄISCHE PATENTSCHRIFT

(11) **EP 2 427 563 B1**
(45) Veröffentlichungstag und Bekanntmachung des Hinweises auf die Patenterteilung: **12.03.2014**
(21) Anmeldenummer: 10716537.5
(22) Anmeldetag: 28.04.2010
(51) Int. Cl.: C12P 7/24, C07C 29/14, C07C 45/66, C07C 51/235

(54) **VERFAHREN ZUR FERMENTATIVEN HERSTELLUNG VON PROPANAL ODER 3-HYDROXYPROPANAL IN GEGENWART VON HYDRAZIDEN, HYDRAZINEN ODER SULFITEN**
PROCESS FOR THE FERMENTATIVE PREPARATION OF PROPANAL OR 3-HYDROXYPROPANAL IN THE PRESENCE OF HYDRAZIDES, HYDRAZINES, OR SULFITES
PROCÉDÉ DE PRÉPARATION FERMENTATIVE DE PROPANAL OU DE 3-HYDROXYPROPANAL EN PRÉSENCE DE HYDRAZIDES, HYDRAZINES, OU SULFITES

(30) Priorität: 05.05.2009 DE 102009002811
(43) Veröffentlichungstag der Anmeldung: 14.03.2012
(73) Patentinhaber: Evonik Degussa GmbH, 45128 Essen (DE)
(72) Erfinder: HAAS, Thomas, 48161 Münster (DE); KLASOVSKY, Florian, 45721 Haltern am See (DE); KRAUTER, Hendrik, 55268 Nieder-Olm (DE); SCHAFFER, Steffen, 45699 Herten (DE); SCHÖBEL, Rene, 58739 Wickede/Ruhr (DE); TACKE, Thomas, 63755 Alzenau (DE); VORLOP, Klaus-Dieter, 38102 Braunschweig (DE); WILLKE, Thomas, 38176 Wendeburg-Bortfeld (DE); WESSEL, Mirja, 44799 Bochum (DE)
(86) Internationale Anmeldenummer: PCT/EP2010/055683
(87) Internationale Veröffentlichungsnummer: WO 2010/127970

(56) Entgegenhaltungen:
- US-A- 4 962 027
- DOLEYRES, Y. ET AL.: "Production of 3-hydroxypropionaldehyde using a two-step process with Lactobacillus reuteri", APPLIED MICROBIOLOGY AND BIOTECHNOLOGY, Bd. 68, Nr. 4, September 2005 (2005-09), Seiten 467-474, XP019331934, in der Anmeldung erwähnt
- KRAUTER, H. ET AL.: "Production of 3-Hydroxypropionaldehyde from glycerol by Lactobacillus reuteri - strong increased biocatalyst lifetime and productivity", JOURNAL OF BIOTECHNOLOGY, Bd. 150, November 2010 (2010-11), Seiten 71-72, XP027489155, [gefunden am 2010-11-01]
- KRAUTER, H. ET AL.: "Production of high amounts of 3-hydroxypropionaldehyde from glycerol by Lactobacillus reuteri with strongly increased biocatalyst lifetime and productivity", NEW BIOTECHNOLOGY, 25. Juni 2011 (2011-06-25), XP000002658362, Gefunden im Internet: URL:http://www.ncbi.nlm.nih.gov/pubmed/217 29774 [gefunden am 2011-09-06]

## Beschreibung

### Gebiet der Erfindung

Gegenstand der Erfindung sind Verfahren zur Herstellung der Aldehyde, 3-Hydroxypropionaldehyd oder Propanal sowie deren Oxidations- und Reduktionsprodukten.

### Stand der Technik

Enzymatische Verfahren zur Herstellung von Aldehyden sind vielfach in der Literatur beschrieben.

So beschreibt zum Beispiel WO2009001304 ein Verfahren zur Herstellung von Aldehyden aus mehrfach ungesättigen Fettsäuren unter Mitwirkung von 13-Hydroperoxid-Lyasen.

Biochemische Routen zur Gewinnung von 3-Methylbutanal werden in Smit et al., Appl. Microbiol. Biotechnol., 2009, 81, 987-999, beschrieben.

Die Herstellung von Aldehyden aus Alkoholen mithilfe von Methanol-Oxidasen und Katalasen aus *Pichia, Hanxenula, Candida,* oder *Torulopsis* ist in US5783429 dargelegt.

Unter Anwendung von Xylol- oder Alkanmonooxygenasen lassen sich gemäß WO2001031047 aromatische Aldehyd-Derivate gewinnen.

3-Hydroxypropionaldehyd (3HPA) lässt sich zu Acrylsäure, einem wichtigen Monomer zur industriellen Herstellung von Polymeren und Kunststoffen, sowie zu ebenso bedeutsamen Acrylsäureestern umsetzen. Weitere bedeutende Produkte, die aus 3-Hydroxypropionaldehyd ableitbar sind, sind 1,3 Propandiol (durch Reduktion), 3-Hydroxypropionsäure (durch Oxidation) und Acrolein (durch Dehydratisierung). Da bisher keine kommerziellen Quellen für 3-Hydroxypropionaldehyd zur Verfügung stehen, wird die Herstellung von 3-Hydroxypropionaldehyd, insbesondere in Form des Reuterins, aus Glycerin intensiv untersucht.

So beschreiben bereits Ende der Achtziger Jahre Talarico und Dobrogosz, Antimicrob. Agents Chemother., 1989, 33, 674-679, die fermentative Herstellung von 3-Hydroxypropionaldehyd aus Glycerin mit *Lactobacillus reuteri.*

Ein analoges Verfahren unter Einsatz von O₂-Limitierung wird in der US5413960 beschrieben.

DK180099 offenbart ein Verfahren mit kontinuierlicher Zufuhr von Glycerin und Einsatz von *Lactobacillus reuteri* DSM12246.

Doleyres et al., Appl Microbiol Biotechnol. 2005 Sep ;68 (4):467-74, beschreiben ein Verfahren zur Herstellung von 3-Hydroxypropionaldehyd in *L. reuteri* ATCC 53608 unter verschiedenen Wachstumsbedingungen und zeigen, dass wiederholter Einsatz des Biokatalysators aufgrund stark fallender Aktivität nicht möglich ist.

CN1778935 beschreibt die Ein-Schritt-Fermentation einer Glycerinlösung mit *Klebsiella pneumoniae* DSM2026 in Anwesenheit von Semicarbazid.

US4962027 beschreibt ein Verfahren zur Herstellung von 3-Hydroxypropionaldehyd in einer aeroben Zwei-Schritt-Fermentation von *Klebsiella pneumoniae* NRRL B-4011, wobei im ersten Schritt zunächst Zellmasse und Glycerin-Dehydratase generiert wird und im zweiten Schritt in Anwesenheit von Semicarbazid Glycerin zu 3-Hydroxypropionaldehyd umgesetzt wird.

EP1669457 beschreibt die biotechnologische Herstellung von 3-Hydroxypropionaldehyd mit (u.a.) *K. pneumoniae* unter Zugabe von Coenzym B₁₂ in nahezu quantitativer Ausbeute durch Erhöhung des Zell/Substrat-Verhältnisses.

Allen beschriebenen Verfahren ist gemein, dass die Ausbeuten und Umsatzraten niedrig sind. Außerdem ist in den beschriebenen Verfahren immer ein hoher Aufwand zur Bereitstellung des Biokatalysators von Nöten, der dann nur eine kurze Standzeit aufweist, das heißt, der anschließend nur kurzzeitig eingesetzt werden kann.

Aufgabe der Erfindung war es daher, ein Verfahren bereitzustellen, welches die beschriebenen Nachteile des Stands der Technik überwindet.

### Beschreibung der Erfindung

Überraschenderweise wurde gefunden, dass die im Folgenden beschriebenen Verfahren die der Erfindung gestellte Aufgabe zu lösen vermögen.

Gegenstand der vorliegenden Erfindung ist daher ein Verfahren zur Herstellung von 3-Hydroxypropionaldehyd oder Propanal umfassend die Verfahrensschritte A) in Kontakt Bringen von Glycerin oder 1,2-Propandiol mit einer Hydro-Lyase ausgewählt aus der Gruppe der Glycerin-Dehydratasen, Diol-Dehydratasen und Propandiol-Dehydratasen, B) Trennung des in Verfahrensschritt A) gebildeten Aldehyds 3-Hydroxypropionaldehyd oder Propanal und der Hydro-Lyase, C) mindestens eine Wiederholung der Verfahrensschritte A) und B) mit der Hydro-Lyase erhalten aus Verfahrensschritt B) und gegebenenfalls D) Isolierung des gebildeten Aldehyds, dadurch gekennzeichnet, dass das Verfahren in Gegenwart einer mit dem Aldehyd 3-Hydroxypropionaldehyd oder Propanal eine Bindung ausbildenden Verbindung ausgewählt aus der Gruppe Hydrazide, Hydrazine, Hydrogen-Sulfite, Sulfite, Metabisulfite und Pyrosulfite durchgeführt wird.

Ein weiterer Gegenstand der Erfindung ist ein Verfahren zur Herstellung von Oxidations- und Reduktionsprodukten des Aldehyds 3-Hydroxypropionaldehyd oder Propanal erhalten durch das vorgenannte Verfahren.

Vorteile des erfindungsgemäßen Verfahrens sind die fast quantitative Ausbeute an Aldehyd bezogen auf den eingesetzten Alkohol, die lange Standzeit der als Biokatalysator eingesetzten Hydro-Lyase und deren Wiederverwendbarkeit. Hierdurch wird eine kontinuierliche Verfahrensweise ermöglicht. Ein weiterer Vorteil des erfindungsgemäßen Verfahrens ist es, dass keine Notwendigkeit zur Unterdrückung von Nebenaktivitäten der als Biokatalysator eingesetzten Hydro-Lyase, wie beispielsweise weitere Reduzierung des Aldehyds zu Alkohol, auf das gebildete Aldehyd besteht.
Noch ein Vorteil der vorliegenden Erfindung ist es, dass das erfindungsgemäße Verfahren die Möglichkeit bietet, zum Großteil unter Ausschluss beziehungsweise Reduzierung von Sauerstoff zu arbeiten, wodurch Korrosion und unerwünschte Oxidation beispielsweise des Produktes vermindert werden kann, ein einfacher apparativer Aufbau ermöglicht wird und Energie eingespart werden kann sowie andere bekannte Probleme der Begasung eines biotechnologischen Reaktors wie beispielsweise Schaumbildung verhindert werden.

Unter dem Begriff "Aldehyd 3-Hydroxypropionaldehyd oder Propanal" werden im Zusammenhang mit der vorliegenden Erfindung ebenfalls Hydrate und Dimere des Aldehyds sowie Mischungen dieser Verbindungen verstanden, sowie beispielsweise Reuterin im Fall des 3-Hydroxypropionaldehyds. Alle angegebenen Prozent (%) sind, wenn nicht anders angegeben, Massenprozent.

Als Hydro-Lyasen können in dem erfindungsgemäßen Verfahren Enzyme ausgewählt aus der Gruppe der Glycerin-Dehydratasen, Diol-Dehydratasen und Propandiol-Dehydratasen, eingesetzt werden.

Hier sind insbesondere solche Hydro-Lyasen für das erfindungsgemäße Verfahren vorteilhaft, die unabhängig von Coenzym B₁₂ aktiv sind, das heißt, die auch ohne Anwesenheit von Coenzym B₁₂ oder Ersatzstoffen / Analoga für diese Substanz wie beispielsweise Corrinoide, Cobalamine, eine Hydro-Lyase-Aktivität aufweisen. Beispielhafte Vertreter solcher Hydro-Lyasen sind der WO2008148640 zu entnehmen.
Die in dem erfindungsgemäßen Verfahren eingesetzten Hydro-Lyasen sind Glycerin-Dehydratasen bzw. Diol-Dehydratasen sowie Propandiol-Dehydratasen (EC 4.2.1.30, EC 4.2.1.28).
In diesem Zusammenhang werden insbesondere die Glycerin-Dehydratasen isolierbar aus Mikroorganismen ausgewählt aus der Gruppe der Gattungen *Klebsiella, Citrobacter, Clostridium, Lactobacillus, Enterobacter, Caloramator, Salmonella* und *Listeria,* besonders bevorzugt die Glycerin-Dehydratase ausgewählt aus der Gruppe der Spezies *Klebsiella pneumoniae, Citrobacter pneumoniae, Clostridium pasteurianum, Lactobacillus leichmannii, Citrobacter intermedium, Lactobacillus reuteri, Lactobacillus buchneri, Lactobacillus brevis, Enterobacter agglomerans, Clostridium pasteurianum, Clostridium perfringens, Clostridium kluyveri, Caloramator viterbensis, Lactobacillus collinoides, Lactobacillus hilgardii, Salmonella typhimurium, Listeria monocytogenes* und *Listeria innocua* in dem erfindungsgemäßen Verfahren eingesetzt.
Die eingesetzte Glycerin-Dehydratase wird vorzugsweise von den Genen kodiert ausgewählt aus der Gruppe bestehend aus *pduC, pduD, pduE, pddA, pddB, pddC, dhaB, dhaC, dhaE* und den gldABC-Genen aus *Lactobacillus reuteri* ATCC55730. Vorteilhaft ist weiterhin der Einsatz der in WO-A-2004/056963 beschriebenen Glycerin-Dehydratase-Variante SHGDH22, welche die in WO2004056963 offenbarte SEQ.-ID-Nr. 322 aufweist. Die Nukleotidsequenz dieser und weiterer geeigneter Gene für eine Glycerin-Dehydratase können beispielsweise der "Kyoto Encyclopedia of Genes and Genomes" (KEGG-Datenbank), den Datenbanken des National Center for Biotechnology Information (NCBI) der National Library of Medicine (Bethesda, MD, USA) oder der Nukleotidsequenz-Datenbank der European Molecular Biologies Laboratories entnommen werden.
Im Zusammenhang mit der Diol-Dehydratase kann es vorteilhaft sein, in dem Verfahren insbesondere die Diol-Dehydratase isolierbar aus Mikroorganismen ausgewählt aus der Gruppe der Gattungen *Klebslella, Propionibacterium, Clostridium, Lactobacillus, Salmonella, Citrobacter, Flavobacterium, Acetobacterium, Brucella* und *Fusobacterium,* besonders bevorzugt isolierbar aus Mikroorganismen ausgewählt aus der Gruppe der Spezies *Klebsiella pneumoniae, Propionibacterium freudenreichii, Clostridium glycolicum, Lactobacillus brevis, Salmonella typhimurium, Citrobacter freundii, Lactobacillus buchneri, Brucella melitensis, Fusobacterium nucleatum, Klebsiella oxytoca, Salmonella typhimurium, Listeria monocytogenes und Listeria innocua* einzusetzen.
Im Zusammenhang mit der Propandiol-Dehydratase kann es vorteilhaft sein, in dem Verfahren insbesondere die Propandiol - Dehydratase isolierbar aus Mikroorganismen ausgewählt aus der Gruppe der Gattungen *Citrobacter, Clostridium, Klebsiella, Lactobacillus, Propionibacterium* und *Salmonella,* besonders bevorzugt isolierbar aus Mikroorganismen ausgewählt aus der Gruppe der Spezies *Citrobacter freundii, Clostridium glycolicum, Klebsiella oxytoca, Klebsiella pneumoniae, Lactobacillus brevis, Lactobacillus buchneri, Lactobacillus collinoides, Propionibacterium freudenreichii* und *Salmonella typhimurium.*

Im Fall, dass die in dem erfindungsgemäßen Verfahren eingesetzte Hydro-Lyase ein Vitamin B₁₂ abhängiges Enzym ist, kann es vorteilhaft sein, Verfahrensschritt A) in Anwesenheit eines Glycerol-Dehydratase-Reaktivase-Aktivität bzw. Diol-Dehydratase-Reaktivase-Aktivität aufweisendes Enzyms bzw. Enzymkomplexes durchzuführen, da die Hydro-Lyase eventuell durch Bindung des Vitamins inaktiviert werden kann. Solch eine Reaktivase ist beispielsweise in Mori et al., J. Biol. Chem. 272:32034 (1997) beschreiben. Bevorzugt werden Reaktivasen eingesetzt, die sich aus dem Organismus isolieren lassen, aus denen sich die korrespondierende Hydro-Lyase isolieren lässt. Geeignete Diol-Dehydratase-Reaktivasen sind bekannt aus *Klebsiella oxytoca* (GenBank Nos: AAC15871, AF017781; GenBank Nos: AAC15872, AF017781) *Salmonella typhimurium* (GenBank Nos: AAB84105, AF026270; GenBank Nos: AAD39008, AF026270) *und Lactobacillus collinoides* (GenBank Nos: CAD01092, AJ297723; GenBank Nos: CAD01093, AJ297723), geeignete Glycerol-Dehydratase-Reaktivase aus *Klebsiella pneumoniae* (vgl. WO2008137403).

Die erfindungsgemäß in dem Verfahren eingesetzten Hydro-Lyasen können in alle dem Fachmann bekannten Formen der Zugänglichkeit eingesetzt werden. So können die eingesetzten Hydro-Lyasen als isolierte Enzyme, beispielsweise aus einem rekombinanten Expressionssystem isoliert, oder als Bestandteil von Enzymkomplexen eingesetzt werden. Bevorzugt eingesetzt werden die Hydro-Lyasen in Form eines Ganzzellreaktors, das heißt, die Hydro-Lyasen liegen in einer biologischen Zelle, bevorzugt in der Zelle, die die Hydro-Lyase expremiert, vor.
Die Hydro-Lyase aufweisenden Zellen können die Hydro-Lyase bereits als Wildtyp exprimieren oder der Art gentechnisch verändert worden sein, dass sie eine Aktivität der Hydro-Lyase erst durch die gentechnische Veränderung aufweisen.
Als Wildtyp Hydro-Lyase enthaltende Zellen können in dem erfindungsgemäßen Verfahren die Zellen eingesetzt werden, die oben beschrieben sind als Mikroorganismen, aus denen die Hydro-Lyasen isolierbar sind.
Gentechnisch veränderte Zellen, die geeignete Hydro-Lyase enthalten und in dem erfindungsgemäßen Verfahren eingesetzt werden können, sind beispielsweise in der Wo2008148640 beschrieben.
Weiterhin kann es von Vorteil sein, die Neigung der Zellen zur reduktiven Bildung von Nebenprodukten aus den Aldehyden, insbesondere die Neigung zur Reduktion der entstehenden Aldehyde zu den korrespondierenden Alkoholen, durch entsprechende gentechnische Modifizierung der Zellen zu vermindern. Beispielsweise können im Falle, dass der mit erfindungsgemäßem Verfahren herzustellende Aldehyd 3-Hydroxypropionaldehyd ist, Zellen mit einer im Vergleich zu ihrem Wildtyp verminderten Aktivität eines Enzyms E₁, welches in der Lage ist, 3-Hydroxypropionaldehyd zu 1,3-Propandiol zu reduzieren, eingesetzt werden. Enzym E₁ (1,3-Propandiol-Dehydrogenase, 3-Hydroxypropionaldehyd-Reduktase, 1,3-Propandiol:NAD⁺-Oxidoreduktase oder Propan-1,3-Diol:NAD⁺-1-Oxidoreduktase; EC 1.1.1.202) katalysiert die Reaktion 3-Hydroxypropionaldehyd + NAD(P)H → 1,3-Propandiol + NAD(P)⁺. Solche Enzyme sind z.B. die Proteine mit den GenBank-Eintragsnummern AP007281.1, EDX43365.1, BAG24545.1, ABQ82306.1, ABO43839.1, EEI08979.1,, EEI66346.1, EEI73647.1, EEJ92522.1, EEI09194.1, EEI73500.1, ABQ83973.1 oder BAG26138.1.

Aus diesen Angaben lassen sich für die jeweilige Hydrolyase enthaltende Zelle, die in erfindungsgemäßem Verfahren eingesetzt wird, entsprechende Oxidoreduktase identifizieren und dessen Aktivität im Vergleich zu ihrem Wildtyp vermindern.
Unter der verwendeten Formulierung "verminderte Aktivität eines Enzyms" wird vorzugsweise eine um einen Faktor von mindestens 0,5, besonders bevorzugt von mindestens 0,1, darüber hinaus bevorzugt von mindestens 0,01, darüber hinaus noch mehr bevorzugt von mindestens 0,001 und am meisten bevorzugt von mindestens 0,0001 verminderte Aktivität verstanden. Die Verminderung der Aktivität eines bestimmten Enzyms kann beispielsweise durch gezielte oder ungerichtete Mutation, durch Zugabe von kompetitiven oder nicht kompetitiven Inhibitoren oder durch andere, dem Fachmann bekannte Maßnahmen zur Verminderung der Synthese eines bestimmten Enzyms erfolgen.
Zur Verminderung der Aktivität eines Enzyms E₁ durch gezielte Mutation kommen beispielsweise gerichtete *knockouts* in Frage, bei denen beispielsweise Promotor aktive Sequenzen des Zielgens, aktive (zur katalytischen Funktion des Enzyms notwendige) Bereiche des Zielgens oder das gesamte Zielgen deletiert, mit Fremd-DNA substituiert oder mit Fremd-DNA unterbrochen werden. Des Weiteren sind dem Fachmann verschiedene Techniken des genesilencing bekannt, die über antisense-Nukleinsäuren oder smallinterfering RNA (siRNA) spezifisch die Transkription bzw. die Translation einzelner Gene vermindern oder gar ganz verhindern. Diese Unterdrückung kann je nach gewähltem System transient erfolgen, induzierbar sein oder konstitutiv vorliegen.
Ebenso sind dem Fachmann Methoden bekannt, mit denen man gezielt einzelne Punktmutationen im Zielgen setzen kann. Weiterhin sind dem Fachmann Methoden, mit dem man ungerichtet Mutationen im Zielgen setzen kann, um im Anschluss Zellen zu selektionieren, die eine verminderte Aktivität eines Enzyms E₁ aufweisen. So können solche Mutationen z.B. durch die natürliche Fehlerrate bei der Replikation des genetischen Materials oder durch Exposition der Zellen gegenüber physikalischen oder chemischen Noxen, wie etwa UV-Licht, N-Methyl-N'-Nitro-N-Nitrosoguanidin, Ethylmethansulfonat, Nitrose Säure, Hydroxyamin oder 4-Nitroquinolin-1-Oxid, ausgelöst werden.

Die erfindungsgemäß in dem Verfahren eingesetzten Hydro-Lyase enthaltenden Zellen können lebende oder tote Zellen sein, wobei lebende Zellen bevorzugt sind.
Es kann vorteilhaft sein, dass die als Hydro-Lyasen eingesetzten, lebenden, Hydro-Lyase enthaltenden Zellen während des Verfahrens in geeignetem Medium gehalten werden, das sie mit den zum Wachstum benötigten Substanzen versorgt. Es hat sich allerdings herausgestellt, dass es hinsichtlich der Versorgung mit Sauerstoff vorteilhaft sein kann, diese zu limitieren. Daher ist es bevorzugt, das die als Hydro-Lyasen eingesetzten, lebenden, Hydro-Lyase enthaltenden Zellen in Verfahrensschritt A) unter mikroanaeroben oder anaeroben Bedingungen eingesetzt werden. Mikroanaerobe oder anaerobe Bedingungen sind bevorzugt dadurch gekennzeichnet, dass das die als Hydro-Lyasen eingesetzten, lebenden, Hydro-Lyase enthaltenden Zellen umgebende Medium einen Gehalt an gelöstem Sauerstoff von kleiner 1 mg/L, bevorzugt von kleiner 0,5 mg/L, insbesondere von kleiner 0,1 mg/L bezogen auf das Gesamtmedium aufweist. Diese Bedingungen können beispielsweise dadurch erreicht werden, dass das die Zellen umgebende Medium nicht zusätzlich durch Luft oder Sauerstoff begast, d.h. durchströmt, wird und gegebenenfalls zusätzlich das den Reaktionsansatz umgebende Behältnis weitestgehend luftdicht abgeschlossen wird.

Um die in Verfahrensschritt B) vorzunehmende Trennung von Aldehyd und Hydro-Lyase vereinfacht durchführen zu können, ist es bevorzugt, dass die Hydro-Lyase auf einem Träger immobilisiert ist. Im Fall, dass die Hydro-Lyase als freies, isoliertes Enzym eingesetzt wird, stehen dem Fachmann viele Methoden der Enzymimmobilisierung zur Verfügung, wie beispielsweise die Kopplung an epoxid-aktivierte Trägermaterialen und in der Literatur beschriebene Verfahren wie zum Beispiel: Mateo et al. Advances in the design of new epoxy supports for enzyme immobilization-stabilization, Biochem Soc Trans. 2007 Dec;35(Pt 6):1593-601 und Balcäo et al., Bioreactors with immobilized lipases: state of the art, Enzyme Microb Technol. 1996 May 1;18(6):392-416.
Im Fall, dass als Hydro-Lyasen Hydro-Lyase-Aktivität aufweisende Zellen eingesetzt werden, können zur Immobilisierung der Zellen dem Fachmann bekannte Verfahren angewendet werden, wie beispielsweise Calciumalginat-Methoden. Insbesondere die in der DE102007031689 beschriebene, auf Siloxanen basierende Methode sowie die Immobilisierung der Zellen mit dem kommerziell erhältliche Lentikat-Liquid ist in diesem Zusammenhang besonders geeignet.

In dem erfindungsgemäßen Verfahren können in Verfahrensschritt A) Glycerin oder 1,2-Propandiol eingesetzt werden.

Es wird Glycerin in dem erfindungsgemäßen Verfahren als vicinales Diol eingesetzt, so dass der erhaltene Aldehyd 3-Hydroxypropionaldehyd ist. Neben Glycerin wird auch 1,2-Propandiol eingesetzt, wobei Propanal als Aldehyd erhalten wird. Dieser Aldehyd kann durch eine sich bereitwillig anschließende Disproportionierung zu n-Propanol und Propionsäure umgewandelt werden.
Das Glycerin oder 1,2-Propandiol wird in Verfahrensschritt A) in einem Konzentrationsbereich von 0,01 Gew.-% bis 50 Gew.-%, bevorzugt von 0,2 Gew.-% bis 20 Gew.-%, bezogen auf die gesamte Reaktionsmischung eingesetzt.

In Verfahrensschritt B) werden der in Verfahrensschritt A) gebildete Aldehyd und die Hydro-Lyase getrennt.
Die Trennung der insbesondere auf einem Träger immobilisierten Hydro-Lyase erfolgt dabei mittels dem Fachmann bekannter Abtrennverfahren, wie beispielsweise Zentrifugation. Denkbar ist in dem Fall, dass in Verfahrensschritt A) als Hydro-Lyase HydroLyase enthaltende Zellen eingesetzt wurden, eine Trennung der Zellen von dem Aldehyd beispielsweise mittels eines geeigneten Filters, etwa mittels eines Filters mit einer Ausschlussgröße in einem Bereich von 20 bis 200 kDa. Denkbar ist auch der Einsatz einer Zentrifuge, einer geeigneten Sedimentationsvorrichtung oder einer Kombination dieser Vorrichtungen, wobei es besonders bevorzugt ist, zumindest einen Teil der Zellen zunächst durch Sedimentation abzutrennen und anschließend den von den Zellen teilweise befreiten Aldehyd einer Ultrafiltration oder Zentrifugationsvorrichtung zuzuführen.

Die auf diese Weise abgetrennten Zellen werden, wenn sie in mehreren nacheinander durchgeführten Trennstufen erhalten wurden, gegebenenfalls vereinigt und können direkt dem Verfahrensschritt A) wieder zugeführt werden. Es kann sich jedoch als vorteilhaft erweisen, die Hydro-Lyase enthaltenden Zellen vor der erneuten Durchführung des Verfahrensschrittes A) noch zu waschen, wobei dieses Waschen vorzugsweise bereits mit demjenigen Medium erfolgt, welches im Verfahrensschritt A) eingesetzt wird.
Besonders bevorzugt erfolgt das Waschen mit dem in Verfahrenschritt A) eingesetzten Medium unter zusätzlicher Zugabe von Glucose; hierdurch kann vorteilhafter Weise während der eigentlichen Biotransformation die Bildung von aus dem Aldehyd abgeleiteten Nebenprodukten unterdrückt werden.
Zum Waschen der Zellen können diese beispielsweise in einem geeigneten Volumen des im Verfahrensschritt A) eingesetzten Mediums resuspendiert und anschließend durch die vorstehend beschriebenen Abtrennverfahren, insbesondere durch Filtration, Sedimentation oder Zentrifugation, oder aber durch eine Kombination dieser Maßnahmen, von dem Medium befreit werden. Analoge Waschvorgänge sind ebenfalls für auf einem Träger immobilisierte Hydro-Lyasen einsetzbar.

Ebenso kann die in Verfahrensschritt B) vorzunehmende Trennung von Aldehyd und Hydro-Lyase dadurch erleichtert werden, dass die mit dem Aldehyd eine Bindung ausbildenden Verbindung auf einem Träger immobilisiert ist. Beispielsweise kommen hier mit Hydrogensulfit beladene Ionenaustauscherharze wie Amberlite® CG400 oder Amberlite® IRA400 (Rohm and Haas) in Frage.

Es ist erfindungswesentlich, dass Verfahrensschritt A) und B) mit der abgetrennten Hydro-Lyase mindestens einmal wiederholt wird, um den Biokatalysator erneut mit frischem Glycerin oder 1,2-Propandiol zur Herstellung des Aldehyds zu nutzen.

In einer bevorzugten, alternativen Ausführungsform des erfindungsgemäßen Verfahrens werden die Verfahrensschritte A), B) und C) kontinuierlich durchgeführt.
Dies ist auf verschiedenen Wegen realisierbar:
Die Hydro-Lyase liegt immobilisiert als stationäre Phase vor, und ein das Glycerin oder 1,2-Propandiol und die mit dem Aldehyd eine Bindung ausbildende Verbindung enthaltende Medium umströmt die immobilisierte Hydro-Lyase und wird gemäß Verfahrensschritt A) mit dieser in Kontakt gebracht. Der entstandene Aldehyd wird als mobile Phase von der stationären Phase abgetrennt, was der Trennung von Aldehyd und Hydro-Lyase in Verfahrensschritt B) entspricht. Das kontinuierlich auf die Hydro-Lyase frisch aufgegebene Glycerin oder 1,2-Propandiol entspricht einer Wiederholung der Verfahrensschritte A) und B) mit der Hydro-Lyase erhalten aus Verfahrensschritt B).
Alternativ kann die mit dem Aldehyd eine Bindung ausbildende Verbindung immobilisiert als stationäre Phase vorliegen. Das Glycerin oder 1,2-Propandiol und die Hydro-Lyase sind in einem Medium enthalten und somit gemäß Verfahrensschritt A) in Kontakt gebracht und umströmen die immobilisierte, mit dem Aldehyd eine Bindung ausbildende Verbindung, wobei der gebildete Aldehyd an der stationären Phase verbleibt. Die Hydro-Lyase verlässt die stationäre Phase, was der Trennung von Aldehyd und Hydro-Lyase in Verfahrensschritt B) entspricht, und wird beispielsweise in Form eines Schlaufenreaktors erneut zusammen mit frischem Glycerin oder 1,2-Propandiol auf die stationäre Phase aufgegeben.
Eine weitere Ausgestaltung eines erfindungsgemäßen Verfahrens, welches kontinuierlich betrieben wird, ist der Art gestaltet, dass sowohl die mit dem Aldehyd eine Bindung ausbildende Verbindung als auch die Hydro-Lyase immobilisiert als stationäre Phasen vorliegen. Ein das Glycerin oder 1,2-Propandiol enthaltende Medium umströmt die stationäre Phase und wird somit mit der Hydo-Lyase gemäß Verfahrensschritt A) in Kontakt gebracht. Der gebildete Aldehyd wird von der mit dem Aldehyd eine Bindung ausbildende Verbindung an die stationäre Phase gebunden und ist somit von der Hydro-Lyase gemäß Verfahrensschritt B) getrennt worden. Das kontinuierliche auf die stationäre Phasen frisch aufgegebene Glycerin oder 1,2-Propandiol entspricht einer Wiederholung der Verfahrensschritte A) und B) mit der Hydro-Lyase erhalten aus Verfahrensschritt B).

Um die gestellte Aufgabe zu lösen, muss während des erfindungsgemäßen Verfahrens eine mit dem Aldehyd eine Bindung ausbildenden Verbindung ausgewählt aus der Gruppe Hydrazide, Hydrazine, Hydrogen-Sulfite, Sulfite, Metabisulfite und Pyrosulfite gegenwärtig sein.

Die mit dem Aldehyd eine Bindung ausbildende Verbindung bildet eine kovalente Bindung mit dem Aldehyd aus. Die mit dem Aldehyd eine Bindung ausbildende Verbindung kann polyfunktionell sein, d.h., dass pro Molekül der Verbindung mehr als eine Bindung zu dem Aldehyd ausgebildet werden kann. Geeignete, mit dem Aldehyd eine Bindung ausbildende Verbindungen sind Hydrazide, Hydrazine, Hydrogen-Sulfite, Sulfite, Metabisulfite und Pyrosulfite, wobei in dem erfindungsgemäßen Verfahren bevorzugt Semicarbazid [CAS 57-56-7], Carbohydrazid [CAS 497-18-7] und Natriumsulfit [CAS 7757-83-7] eingesetzt werden.

Diese können frei in Lösung aber auch immobilisiert auf Trägern eingesetzt werden, bevorzugt sind in diesem Zusammenhang nicht immobilisierte, gelöste Verbindungen, da diese effektiver und schneller mit dem Aldehyd eine Bindung eingehen können und somit unerwünschte Nebenreaktionen verhindern können.
Im dem Fall, dass das erfindungsgemäße Verfahren mit Zellen durchgeführt wird, ist es erfindungsgemäß bevorzugt, wenn die mit dem Aldehyd eine Bindung ausbildenden Verbindung in das die Zelle umgebende Medium gegeben wird.
Auch Mischungen der mit dem Aldehyd eine Bindung ausbildenden Verbindungen können eingesetzt werden.
Es ist bevorzugt, wenn in dem erfindungsgemäßen Verfahren das Glycerin oder 1,2-Propandiol und die funktionelle Gruppe der mit dem Aldehyd eine Bindung ausbildenden Verbindung in einem molaren Verhältnis von mindestens 1:1, bevorzugt von mindestens 1:1,2, insbesondere von mindestens 1:1,5 eingesetzt werden.

Im Verfahrensschritt D) des erfindungsgemäßen Verfahrens können die gebildeten Aldehyde isoliert werden, wobei zur Isolierung alle dem Fachmann bekannten Methoden zur Isolierung von niedermolekularen Substanzen aus komplexen Zusammensetzungen in Betracht kommen. Beispielhaft seien an dieser Stelle die Fällung mittels geeigneter Lösungsmittel, die Extraktion mittels geeigneter Lösungsmittel, die Komplexierung, beispielsweise mittels Cyclodextrinen oder Cyclodextrin-Derivaten, die Kristallisation, die Aufreinigung bzw. Isolierung mittels chromatographischer Methoden oder die Überführung der Aldehyde in leicht abtrennbare Derivate genannt.
Teil der Isolierung in Verfahrensschritt D) kann auch sein, die mit dem Aldehyd eine Bindung ausbildende Verbindung wieder vom Aldehyd zu trennen.

Dies lässt sich durch dem Fachmann an sich bekannte Methoden in Abhängigkeit der vorliegenden Bindung erreichen. So können beispielsweise Bindungen zwischen Aldehyden und Semicarbazid oder Carbohydrazid durch Säure gespalten werden; Bindungen zwischen dem Komplex aus Aldehyd und der mit dem Aldehyd eine Bindung eingehenden Verbindung sowie Amberlite® CG400 oder Amberlite® IRA400 können beispielsweise durch Erhöhen der Salzkonzentrationen, etwa von 1M NaCl oder einem System aus NaHCO₃/Na₂CO₃ (0,15 M/0,075 M) getrennt werden. Des Weiteren kann eine Bindung zwischen dem Aldehyd und der mit dem Aldehyd eine Bindung eingehenden Verbindung durch eine thermische Behandlung gelöst werden.

Einen weiteren Beitrag zur Lösung der eingangs genannten Aufgaben leistet auch ein Verfahren zur Herstellung von Oxidations- oder Reduktionsprodukten eines Aldehyds, umfassend die Verfahrensschritte:
I) Bereitstellung eines Aldehyds mittels des vorstehend beschriebenen Verfahrens zur Herstellung von 3-Hydroxypropionaldehyd oder Propanal aus Glycerin oder 1,2-Propandiol,
II) chemische oder biokatalytische Umsetzung des Aldehyds durch Reduktion, Oxidation, Disproportionierung oder Dehydratisierung unter Erhalt von gegebenenfalls ungesättigten Reduktions- oder Oxidationszwischenprodukten und gegebenenfalls
III) die weitere chemische oder biokatalytische Umsetzung der im Verfahrensschritt II) erhaltenen Reduktions- oder Oxidationszwischenprodukte durch Reduktion, Oxidation oder Addition.
Oxidations- oder Reduktionsprodukte des Aldehyds umfassen Verbindungen wie beispielsweise gegebenenfalls ungesättigte Carbonsäuren, Carbonsäurederivate, Amine, Isocyanate, Acetale, Nitrile und Oxime.

Die Reduktions- oder Oxidationszwischenprodukte des Verfahrensschrittes II) können daher bereits die gewünschten Reduktions- oder Oxidationsprodukte des Aldehyds darstellen.

Zunächst wird im Verfahrensschritt I) ein Aldehyd mittels des vorstehend beschriebenen Verfahrens zur Herstellung von Aldehyden bereitgestellt, wobei dieses gegebenenfalls, wie vorstehend im Zusammenhang mit diesen Verfahren beschrieben, aufgereinigt worden sein kann.
Im Verfahrensschritt II) kann der Aldehyd dann chemisch oder biokatalytisch durch Reduktion, Oxidation, Disproportionierung oder Dehydratisierung unter Erhalt von Reduktions- oder Oxidationszwischenprodukten, insbesondere im Fall, dass der in Verfahrensschritt I) erhaltene Aldehyd 3-Hydroxypropionaldehyd ist, unter Erhalt von 1, 3-Propandiol (1,3-PDO) (Reduktion), Acrolein (Dehydratisierung) oder 3-Hydroxypropionsäure (Oxidation), umgesetzt werden. Dabei sind Einzelheiten zur Umsetzung von 3-Hydroxypropionaldehyd zu Acrolein unter anderem durch Hall und Stern in Journal of the Chemical Society, 1950, Seiten 490-498 beschrieben, während Einzelheiten zur Herstellung von 1, 3-Propandiol aus 3-Hydroxypropionaldehyd unter anderem der US 5,334,778 entnommen werden können. Die Herstellung von 3-Hydroxypropionsäure aus 3-Hydroxypropionaldehyd wiederum ist unter anderem in US 6,852,517 beschrieben. Im Fall, dass der in Verfahrensschritt I) erhaltene Aldehyd Propanal aus 1,2-Propandiol darstellt, dann können 1-Propanol und Propionsäure durch bereitwillige Disproportionierung des Propanals im Biotranformationsmedium entstehen (Sriramulu, D. D.; Liang, M.; Hernandez-Romero, D.; Raux-Deery, E.; Lunsdorf, H.; Parsons, J. B.; Warren, M. J. & Prentice, M. B. J. Bacteriol., 2008, 190, 4559-4567).

Gegebenenfalls können die im Verfahrensschritt II) erhaltenen Reduktions- oder Oxidationszwischenprodukte in einem weiteren Verfahrensschritt III) chemisch oder mikrobiologisch durch Reduktion, Oxidation oder Addition noch weiter umgesetzt werden. In Betracht kommt hier insbesondere die Umsetzung von im Verfahrensschritt II) erhaltenem Acrolein durch Oxidation zur Acrylsäure, die dann gegebenenfalls in einem noch weiteren Verfahrensschritt IV) radikalisch unter Bildung von auf Acrylsäure basierenden Polymeren umgesetzt werden kann. In Betracht kommt hier insbesondere die radikalische Polymerisation von gegebenenfalls teilneutralisierter Acrylsäure in Gegenwart geeigneter Vernetzer unter Bildung wasserabsorbierender Polyacrylate, die auch als "Superabsorber" bezeichnet werden. Die chemische Oxidation von Acrolein zu Acrylsäure und die anschließende radikalische Polymerisation der so erhaltenen Acrylsäure ist unter anderem in der WO-A-2006/136336 beschrieben.
Weiterhin kommt es ebenfalls in Betracht, dass die Umsetzung von im Verfahrensschritt II) erhaltenem Acrolein durch oxidative Veresterung zu Acrylsäureestern, die dann gegebenenfalls in einem noch weiteren Verfahrensschritt IV) radikalisch unter Bildung von auf Acrylsäureestern basierenden Polymeren umgesetzt werden können.

Bevorzugte Reduktions- oder Oxidationsprodukte des Aldehyds erhältlich aus dem erfindungsgemäßen Verfahren über oben dargelegte Schritte stellen somit die Substanzen 1,3-Propandiol, 1-Propanol, Acrolein, 3-Hydroxypropionsäure, Propionsäure, Acrylsäure, Acrylsäureester und Polymere basierend auf Acrylsäure oder Acrylsäureestern dar, wobei der in Verfahrensschritt I) erhaltene Aldehyd 3-Hydroxypropionaldehyd ist.

In den nachfolgend aufgeführten Beispielen wird die vorliegende Erfindung beispielhaft beschrieben.
Folgende Figuren sind Bestandteil der Beispiele:
Figur 1: Konzentrationsverlauf von Glycerin, 3HPA und 1,3-PDO bei Umsetzung von Glycerin mit immobilisierten *L. reuteri*-Zellen im Verlauf eines Biotransformations-Zyklus.
Figur 2: Konzentrationsverlauf von Glycerin, 3HPA und 1,3-PDO bei Umsetzung von Glycerin mit immobilisierten *L. reuteri*-Zellen im Verlauf von 7 Biotransformations-Zyklen und i. Ggw. von Semicarbazid als Fängerstoff.
Figur 3: Akkumulierte Ausbeute von 3HPA bei Umsetzung von Glycerin mit immobilisierten *L. reuteri*-Zellen im Verlauf von 7 Biotransformations-Zyklen und i. Ggw. von Semicarbazid als Fängerstoff.
Figur 4: Konzentrationsverlauf von Glycerin, 3HPA und 1,3-PDO bei Umsetzung von Glycerin mit immobilisierten *L. reuteri*-Zellen im Verlauf von 10 Biotransformations-Zyklen und i. Ggw. von Carbohydrazid als Fängerstoff.
Figur 5: Akkumulierte Ausbeute von 3HPA bei Umsetzung von Glycerin mit immobilisierten *L. reuteri*-Zellen im Verlauf von 10 Biotransformations-Zyklen und i. Ggw. von Carbohydrazid als Fängerstoff.
Figur 6: zeitlicher Verlauf der Konzentrationen von Glycerin und 1,2-Propandiol bei Biotransformation von Glycerin bzw. von 1,2-Propandiol mit L.reuteri-Zellen
Figur 7: Verlauf der Konzentrationen von 3HPA, Glycerin und 1,3-PDO bei sieben aufeinanderfolgenden Biotransformationen von Glycerol mit immobilisierten L. reuteri-Zellen ohne Zusatz von Sulfit (Vergleichsbeispiel)
Figur 8: Verlauf der Konzentrationen von 3HPA, Glycerin und 1,3-PDO bei sieben aufeinanderfolgenden Biotransformationen von Glycerol mit immobilisierten *L*. *reuteri*-Zellen mit Zusatz von Natriumhydrogensulfit (erfindungsgemäß).
Figur 9: zeitlicher Verlauf der Konzentrationen von 3HPA, Glycerin und 1,3-PDO bei Biotransformation von Glycerin mit Wildtyp *L.reuteri*-Zellen
Figur 10: zeitlicher Verlauf der Konzentrationen von 3HPA, Glycerin und 1,3-PDO bei Biotransformation von Glycerin mit gentechnisch modifizierten *L*. *reuteri*-Zellen.

### Beispiele:

### Beispiel 1: Kultivierung von Lactobacillus reuteri

Die Kultivierung von *Lactobacillus reuteri* erfolgte in anaerobisiertem MRS Medium (Medium für *Lactobacillus* nach DeMan, Rogosa und Sharpe) mit 20 mM Glycerin-Zusatz (MRSG20) in verschlossen geeigneten Gebinden bei 80 rpm. Das Medium MRSG20 (Zusammensetzung [Angaben pro Liter]: 10 g Proteose Pepton Nr. 3 (Roth), 8 g Rinderextrakt (Roth), 4 g Hefeextrakt (VWR), 1.5 mL Glycerin (Roth), 1 mL Tween80 (Roth), 40 mL Salzlösung [10 g/L Dikaliumhydrogenphosphat {Roth}, 250 g/L Natriumacetat-Trihydrat {Roth}, 100 g/L Diammoniumhydrogencitrat {Roth}, 10 g/L Magnesiumsulfat-Heptahydrat {Roth}, 2.5 g/L Mangansulfat-Tetrahydrat {Roth}] wurde nach Anaerobisieren und Autoklavieren (20 min bei 121 °C) mit 25 mL einer Glucose-Lösung (4 mol/L) versetzt.
Für die Vorkultur 1 (50 mL) wurde eine Kryo-Kultur verwendet. Hierfür wurde *L. reuteri* SD2112 in MRS-Medium, welches zusätzlich 6 % (w/v) Magermilchpulver und 10 % (v/v) Glycerin enthält, bei -80 °C gelagert. Zur Herstellung der stock culture wird eine Übernachtkultur (10 ml MRS-Medium) 1:1 mit der stock Lösung gemischt. Die Vorkultur diente als 1%iges Inokulum und wurde bei 33°C inkubiert. Nach 15 Stunden wurde aus diesem Ansatz eine zweite Vorkultur (50 mL) 1%ig angeimpft und 9 Stunden bei 37°C inkubiert. Diese diente wiederum als 1%iges Inokulum für die Hauptkultur (2-mal 1L). Nach 15 Stunden bei 33°C wurden die Zellen geerntet.
Hierfür wurden 2 Liter abzentrifugiert (4000 g; 20°C; 10 min), der Überstand verworfen und beide resultierenden Pellets in 1 Liter 0.1 M Kaliumphosphat-Puffer (KPP; K₂HPO₄ + KH₂PO₄ [Roth]) bei pH 7 resuspendiert. Es erfolgte eine erneute Zentrifugation unter gleichen Bedingungen. Der Überstand wurde erneut verworfen, das Zell-Pellet in 0.1 M KPP bei pH 7 im Verhältnis 1 g zu 1 mL resuspendiert und unter N₂ gelagert. Diese Suspension wurde für die anschließende Immobilisierung genutzt.

### Beispiel 2: Immobilisierung von Lactobacillus reuteri

Zur Immobilisierung wurden 20 mL der in Beispiel 1 beschriebenen Zellsuspension mit auf ∼35°C temperierter Lentikat-Liquid^{®} (GeniaLab) vermischt, die Lentikats mit dem LentiKat^{®}-Printer (GeniaLab) hergestellt und auf eine Restfeuchte von 28% getrocknet. Nach der Rückquellung in LentiKat-Stabilizer® (GeniaLab) wurden die Immobilisate für mind. 2 Stunden in anoxischer Atmosphäre bei 300 rpm zur Stabilisierung der Lentikats in LentiKat-Stabilizer® gerührt. Die abgetrennten und zweimal mit 0.1 M KPP bei pH 7 gewaschenen Immobilisate wurden 3%ig für 15 Stunden bei 33°C stehend in MRSG20 in einer voll gefüllten 1-L-Flasche mit Möglichkeit zum Gasaustausch regeneriert. Nach Trennung der Immobilisate vom Medium (aerobe Bedingungen) wurden diese zweimal mit 0.1 M KPP (pH 7) gewaschen.

### Beispiel 3: HPLC-basierte Quantifizierung von Glycerin und 1,3-Propandiol

Zur Quantifizierung von Glycerin und 1,3-Propandiol (1,3-PDO) wurde in je 1 ml Zellsuspension die Biomasse durch Zentrifugation (10 min, 16.100 g, 4°C) abgetrennt und 40 µl des Kulturüberstands unter Verwendung eines Shimadzu-HPLC-Systems (Autosampler SIL-10AT, Pumpe LC-10AT, Degasser DGU-3A, Brechungsindex-Detektor RID-10A, UV-Detektor SPD-10A, Ofen CTO-10A, Controller SCA-10A-VP) mit einer Aminex HPX-87H 300 x 7.8 mm Trennsäule (Bio-Rad Laboratories GmbH, München) mit 9 µm Partikelgröße und unter Verwendung einer Vorsäule (HPX-87H, 30 x 4.6 mm) analysiert. Die zu analysierenden Substanzen wurden isokratisch mit einer mobilen Phase aus 5 mM Schwefelsäure mit einer Flussrate von 0,6 ml/min 20 min bei 40°C eluiert. Die Identifizierung erfolgte durch einen Vergleich mit der Verweilzeit von Referenzsubstanzen (Sigma-Aldrich Chemie GmbH, Steinheim). Die Konzentration der Verbindungen in den Kulturüberständen wurde über einen Vergleich der Peakflächen mit einer zuvor mit externen Standards erstellten Kalibriergerade berechnet.

### Beispiel 4: Umsetzung von Glycerin zu 3HPA mit immobilisierten Lactobacillus reuteri (Vergleichsbeispiel)

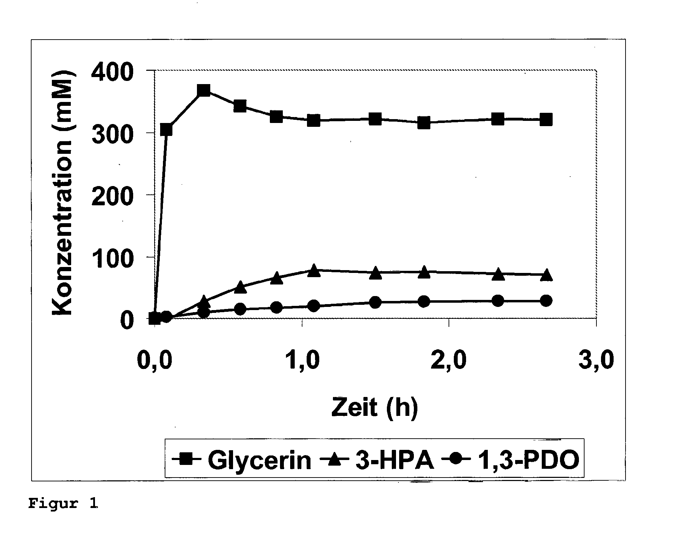

Die gemäß Beispiel 2 hergestellten, mit *L. reuteri* beladenen Immobilisate wurden 10%ig (20 g, entsprechend einer Konzentration von 2 g Trockenzellen pro Liter) in einer Biotransformation in einem Glasfermenter (Gesamtvolumen 500 mL) mit 200 mL Reaktionspuffer eingesetzt. Letzterer bestand aus anaerobisiertem und auf 35°C vortemperiertem 0.1 M KPP (pH 7). Nach der Einstellung eines konstanten pH-Wertes von 7 und einer konstanten Temperatur von 35 °C wurde die Reaktion durch Zugabe von 8 mL 98%igem Glycerin (Roth, entsprechend einer Konzentration von 500 mM in der Reaktionsmischung) gestartet. Die Beprobung erfolgte für eine Stunde alle 10 Minuten und nachfolgend für 2 Stunden alle 30 Minuten. Die Konzentrationen von Glycerin und der Nebenprodukte wurden mittels HPLC gemäß Beispiel 3 bestimmt.
Eine Quantifizierung von 3-Hydroxypropionaldehyd erfolgte mittels eines colorimetrischen Tests gemäß Doleyres et al. ("Production of 3-hydroxypropionaldehyde using a two-step process with Lactobacillus reuteri, Applied Microbiology and Biotechnology, Vol. 68, 2005, Seiten 467-474).
Nach einer Reaktionszeit von ∼100 Minuten konnte sowohl im Falle der freien wie auch im Falle der immobilisierten Zellen kein Umsatz von Glycerin mehr beobachtet werden. Konzentrationsverläufe für Glycerin, 3HPA und 1,3-PDO sind in Figur 1 wiedergegeben; während der Biotransformation wurden 1.29 g 3HPA erzeugt. Das Reaktionsmedium wurde von den Zellen bzw. Immobilisaten abgetrennt und separat bei 4°C gelagert.
Die Umsetzung von Glycerin zu 3HPA und den Nebenprodukten erfolgte gemäß der vorstehend beschriebenen Analytik zu ∼25%. Bei Wiederverwendung der Zellen bzw. Immobilisate konnte kein Umsatz von Glycerin zu 3HPA festgestelt werden.

### Beispiel 5: Umsetzung von Glycerin zu 3HPA mit immobilisierten Lactobacillus reuteri und in Gegenwart von Semicarbazid, erfindungsgemäß

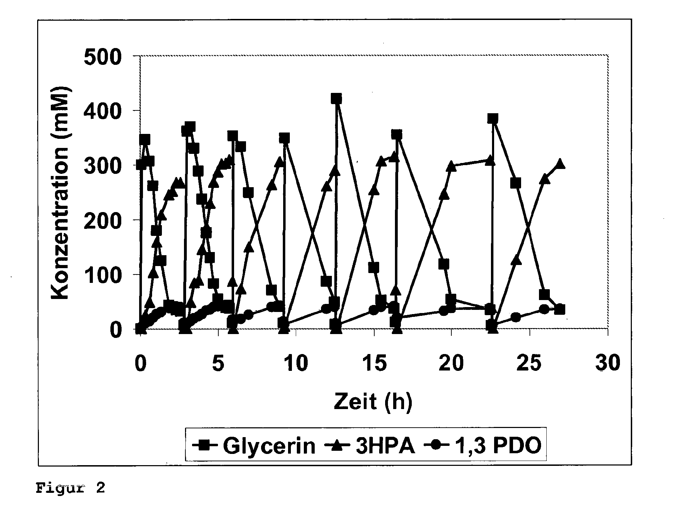

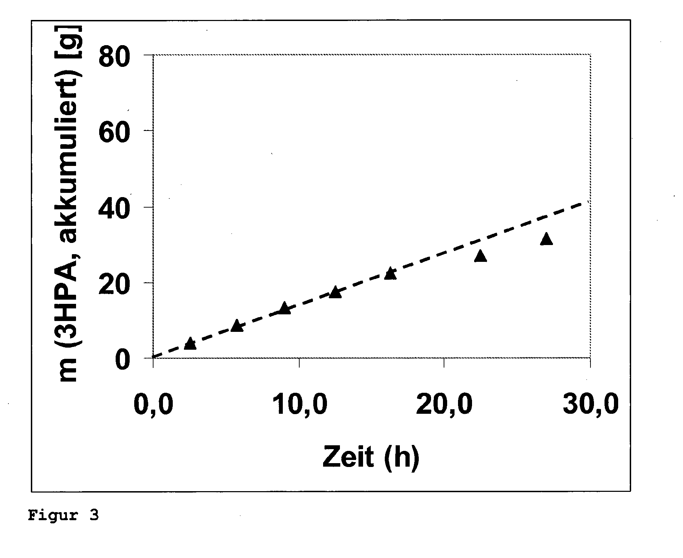

Die gemäß Beispiel 2 hergestellten, mit *L. reuteri* beladenen Immobilisate wurden 10%ig (20 g, entsprechend einer Konzentration von 2 g Trockenzellen pro Liter) in einer Biotransformation in einem Glasfermenter (Gesamtvolumen 500 mL) mit 200 mL Reaktionspuffer eingesetzt. Dieser besteht aus 0.1 M KPP (pH 7) sowie 500 mM Semicarbazid und wurde anaerobisiert sowie auf 35°C Reaktionstemperatur vortemperiert. Nach Einstellung eines konstanten pH-Wertes von 7 und einer konstanten Temperatur von 35 °C wurde die Reaktion durch Zugabe von 8 mL 98%igem Glycerin (Roth; entsprechend einer Konzentration von 500 mM in der Reaktionsmischung) gestartet. Die Entnahme und Analyse von Proben aus der Reaktionsmischung erfolgte wie in Beispiel 4 beschrieben.
Nach einer Reaktionszeit von 150 Minuten wurde das Reaktionsmedium von den Immobilisaten abgetrennt und neues anaerobisiertes, vortemperiertes Reaktionsmedium (0,1 M KPP pH 7 mit 500 mM Semicarbazid) zugegeben. Wie zuvor beschrieben wurde ein weiterer Biotransformationszyklus durchgeführt. Insgesamt wurden 7 aufeinanderfolgende Zyklen ohne zwischenzeitliche Lagerung der Immobilisate durchgeführt. Konzentrationsverläufe für Glycerin, 3HPA und 1,3-PDO sind in Figur 2 wiedergegeben; Figur 3 zeigt die akkumulierten Ausbeuten an 3HPA.
Die Umsetzung von Glycerin zu 3HPA erfolgte mit Semicarbazid als Fängerstoff gemäß der vorstehend beschriebenen Analytik in den ersten 5 Zyklen nur zu ∼55%; in den weiteren 2 Zyklen verringerte sich die Ausbeute kontinuierlich.

### Beispiel 6: Umsetzung von Glycerin zu 3HPA mit immobilisierten Lactobacillus reuteri in Gegen wart von Carbohydrazid, erfindungsgemäß

Die gemäß Beispiel 2 hergestellten, mit *L. reuteri* beladenen Immobilisate wurden 10%ig (20 g, entsprechend einer Konzentration von 2 g Trockenzellen pro Liter) in einer Biotransformation in einem Glasfermenter (Gesamtvolumen 500 mL) mit 200 mL Reaktionspuffer eingesetzt. Dieser besteht aus 0.1 M KPP (pH 7) sowie 520 mM Carbohydrazid und wurde anaerobisiert sowie auf 35°C Reaktionstemperatur vortemperiert. Nach Einstellung eines konstanten pH-Wertes von 7 und einer konstanten Temperatur von 35 °C wurde die Reaktion durch Zugabe von 8 mL 98%igem Glycerin (Roth; entsprechend einer Konzentration von 500 mM in der Reaktionsmischung) gestartet. Die Entnahme und Analyse von Proben aus der Reaktionsmischung erfolgte wie in Beispiel 4 beschrieben.

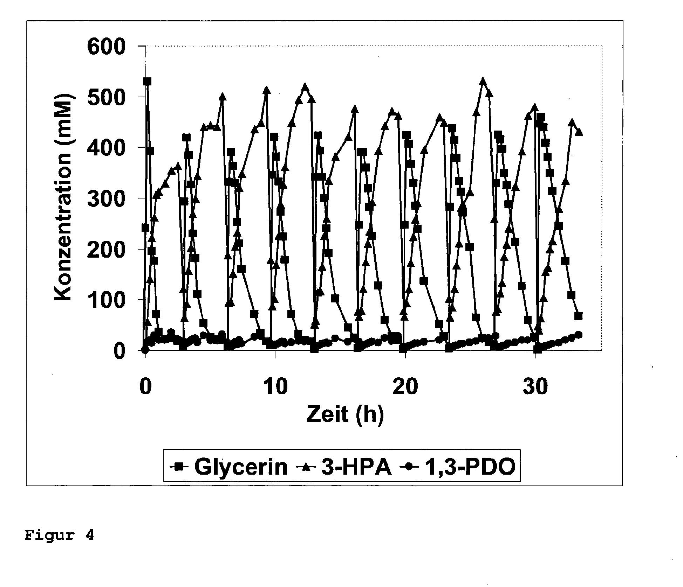

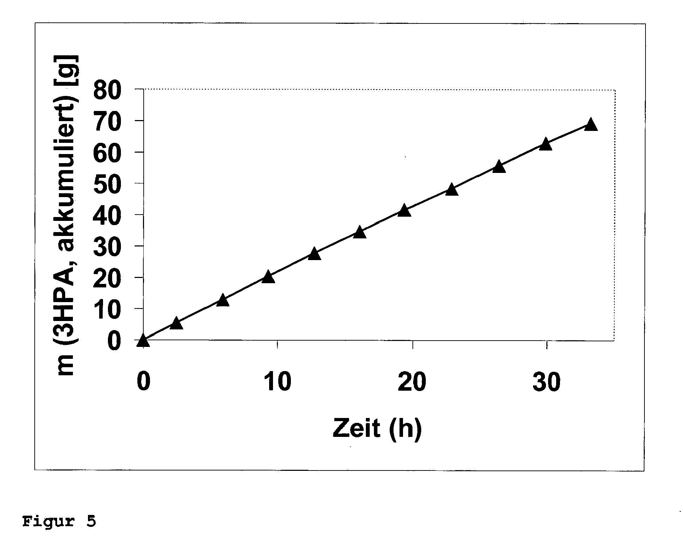

Nach einer Reaktionszeit von 180 Minuten wurde das Reaktionsmedium von den Immobilisaten abgetrennt und neues anaerobisiertes, vortemperiertes Reaktionsmedium (0,1 M KPP pH 7 mit 520 mM Carbohydrazid) zugegeben. Wie zuvor beschrieben wurde ein weiterer Biotransformationszyklus durchgeführt. Insgesamt wurden 10 aufeinander folgende Zyklen ohne zwischenzeitliche Lagerung der Immobilisate durchgeführt; Konzentrationsverläufe für Glycerin, 3HPA und 1,3-PDO sind in Figur 4 wiedergegeben; Figur 5 zeigt die akkumulierten Ausbeuten an 3HPA.
Die Umsetzung von Glycerin zu 3HPA und Nebenprodukten erfolgte mit Carbohydrazid als Fängerstoff gemäß der vorstehend beschriebenen Analytik in allen 10 Zyklen zu ∼95%.

### Beispiel 7: Umsetzung von Glycerin zu 3HPA mit freien Lactobacillus reuteri in Gegenwart von schwefelhaltigen Verbindungen, erfindungsgemäß

Die gemäß Beispiel 1 hergestellten Zellen wurden in einer 1000 millimolaren Glycerinlösung suspendiert, so daß eine Gesamt-Zellkonzentration von 2.2x10¹⁰ CFU/ml resultiert. Nach Zugabe einer schwefelhaltigen Verbindung (Natriumsulfit, Natriumhydrogensulfit oder Natriumpyrosulfit; jeweils mit einer Endkonzentration von 100 mmol/L in der Reaktionslösung) wurde die Suspension für 120 min inkubiert, wobei die Mischung durch Einleiten eines kontinuierlichen Stickstoffstroms sauerstofffrei gehalten wurde. Die in Tabelle 1 aufgelisteten, nach Ablauf von 120 min resultierenden Konzentrationen von 3HPA und 1,3-PDO sowie die Zell-Viabilitäten verdeutlichen die Erhöhung der erzielbaren 3-HPA-Konzentration sowie die Reduktion der Abnahme der Zell-Viabilitäten durch Zugabe der schwefelhaltigen Verbindungen.

**Tabelle 1**

| Schwefelhaltige Verbindung | c (3-HPA) [mmol/L] | c (1,3-PDO) [mmol/L] | Zell-Viabilität [CFU/mL] |
|---|---|---|---|
| ohne | 504 | 32 | 5.1*10⁴ |
| Na₂S₂O₅ | 524 | 31 | > 6.0*10⁹ |
| NaHSO₃ | 546 | 30 | 3.2*10⁹ |
| Na₂SO₃ | 607 | 31 | 2.6*10⁹ |

### Beispiel 8: Umsetzung von 1,2-Propandiol bzw. Glycerin zu Propanal bzw. 3-Hydroxypropionaldehyd mit immobilisierten Lactobacillus reuteri in Gegenwart von Carbohydrazid, erfindungsgemäß

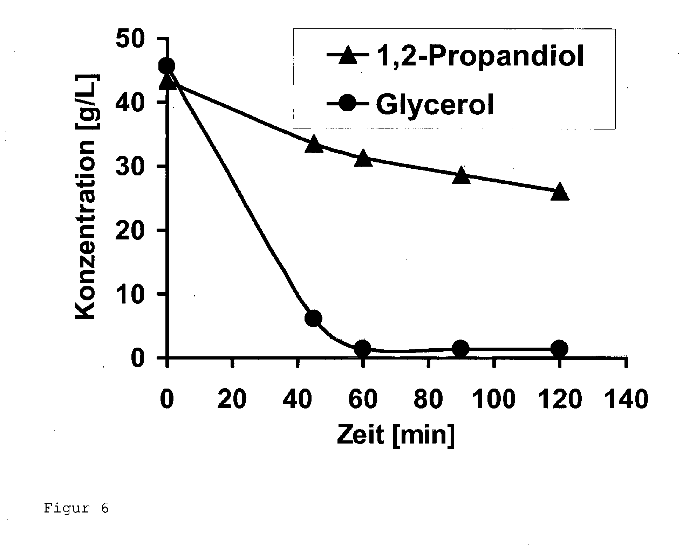

Die gemäß Beispiel 1 hergestellten Zellen wurden je in einer 500 millimolaren Glycerinlösung bzw. einer 500 millimolaren 1,2-Propandiollösung suspendiert, so daß eine Gesamt-Zellkonzentration von 2.2x10¹⁰ CFU/ml resultiert. Nach Zugabe von Carbohydrazid mit einer Endkonzentration von 100 mmol/L in der Reaktionslösung wurde die Suspension für 120 min inkubiert, wobei die Mischung durch Einleiten eines kontinuierlichen Stickstoffstroms sauerstofffrei gehalten wurde. Die in Figur 6 dargestellten Konzentrationsverläufe von Glycerin und 1,2-Propandiol verdeutlichen die gleichwertige Transformation der beiden Substrate.

### Beispiel 9: Umsetzung von Glycerin zu 3HPA mit immobilisierten Lactobacillus reuteri Zellen in Gegenwart von schwefelhaltigen Verbindungen und unter Zufügung von Glucose zur Waschlösung, erfindungsgemäß

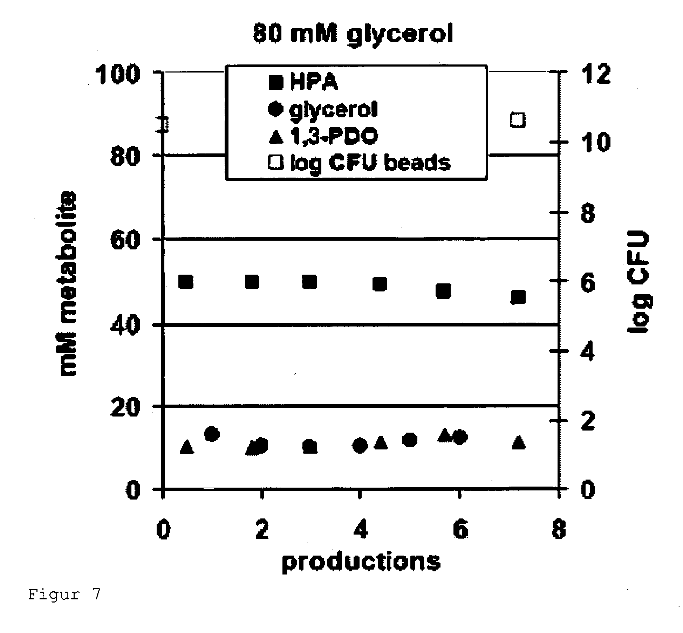

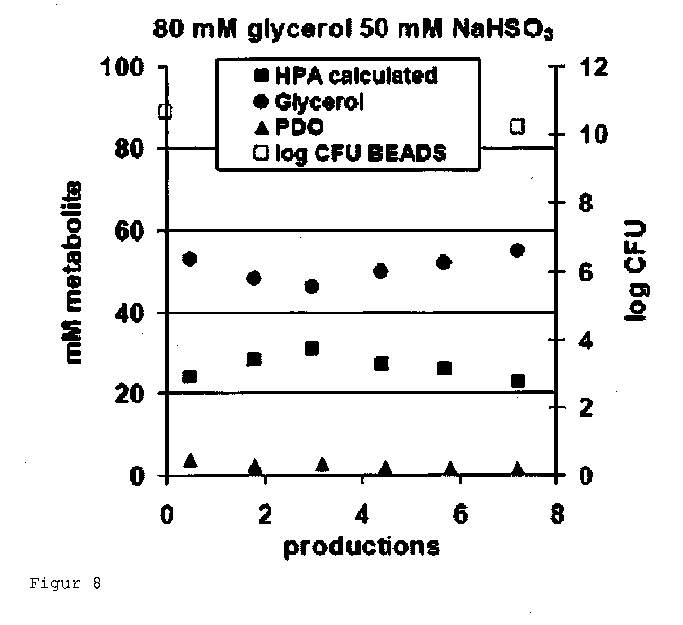

Die gemäß Beispiel 2 hergestellten, mit *L. reuteri* beladenen Immobilisate wurden 20%ig (4 mL) in einer Biotransformation in einem Glasfermenter mit 20 mL Reaktionslösung eingesetzt. Diese enthält 80 mmol/L Glycerin und wurde nach Temperieren auf 30°C auf einen konstanten pH-Wert von 5 eingestellt (Vergleichs- und erfindungsgemäßes Beispiel). Im erfindungsgemäßen Ansatz wurde der Reaktionslösung zusätzlich Natriumhydrogensulfit (gemäß einer Endkonzentration von 50 mmol/L) zugesetzt. Nach Ablauf eines Biotransformationszyklus (30 min) werden die Immobilisate abfiltriert, für die Dauer von 30 min. in MRSG20 (s. Beispiel 1) regeneriert und erneut wie beschrieben in einer Reaktionslösung eingesetzt. Auf die beschriebene Weise werden insgesamt 7 Umsetzungen von Glycerin durchgeführt. Während ohne Zusatz von Hydrogensulfit in jedem Tranformationsschritt 10 mmol/L 1,3-PDO gebildet werden (Figur 7), kann bei Zusatz von Natriumhydrogensulfit eine Reduzierung der 1,3-PDO-Konzentration beobachtet werden (Figur 8).

### Beispiel 10: Umsetzung von Glycerin zu 3HPA mit freien, gentechnisch unmodifizierten bzw. modifizierten Lactobacillus reuteri.

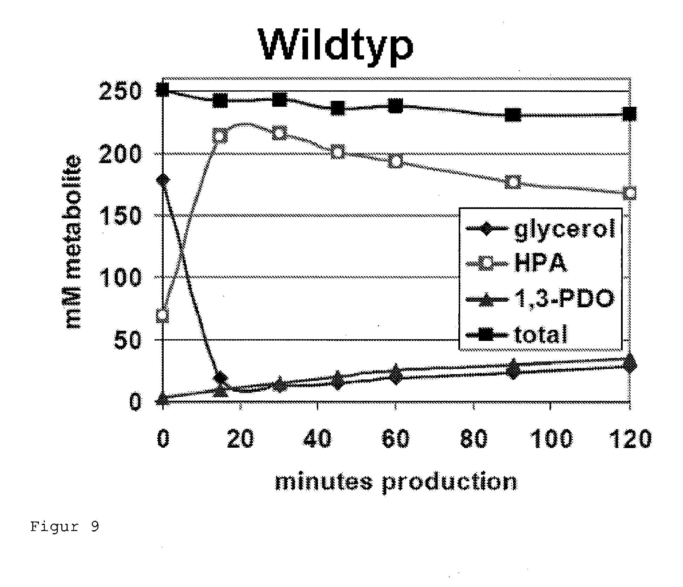

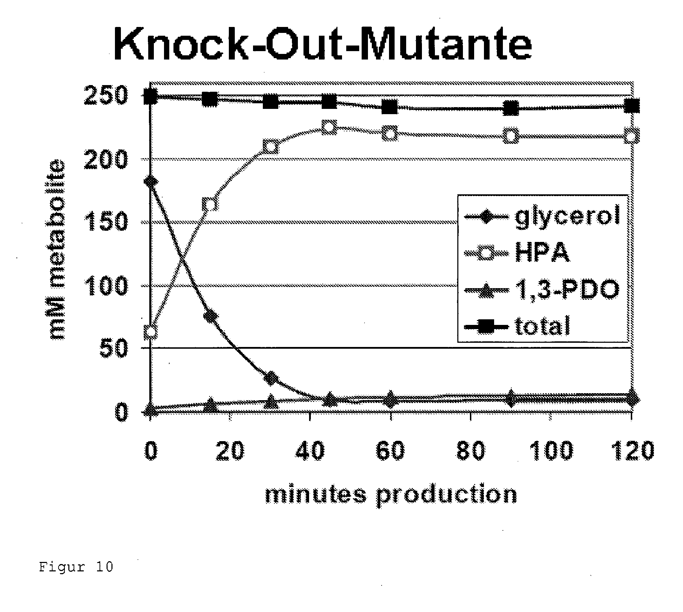

Die Wildtyp-Zellen bzw. die durch Deletion des Oxidoreduktase-Gens generierten Zellen wurden in einer 250 millimolaren Glycerinlösung suspendiert, so daß jeweils eine Gesamt-Zellkonzentration von 1.2x10¹⁰ CFU/ml resultiert. Wie die Konzentrationsverläufe im Zuge einer 120-minütigen Biotransformation zeigen, wird bei Einsatz des *L. reuteri-*Wildtyps das nach 20 minütiger Biotransformation gebildete 3HPA zugunsten der Bildung von 1,3-PDO abgebaut (Figur 9), während die Umwandlung von 3HPA zu 1,3-PDO bei Verwendung der durch Deletion des Oxidoreduktase-Gens generierten Zellen verhindert wird (Figur 10).

### Beispiel 11: Herstellung von Acrolein aus einem 3HPA enthaltenden Fermentationsaustrag, erfindungsgemäß

Aus den gemäß Beispiel 4 - 6 erhaltenen Fermentations-Austrägen konnte Acrolein durch saure Hydrolyse bei erhöhten Temperaturen gewonnen werden. Hierzu wurde ein Teil des Fermentationsaustrages im Verhältnis 1:100 mit Wasser verdünnt, mit 3 Teilen 37%iger Salzsäure vermischt und 0,5 h bei 37 °C inkubiert. Das hierbei gebildete Acrolein bildete nach Zugabe einer DL-Tryptophan-Lösung (Fluka, 0.01 mol/L Tryptophan in 0.05 mol/L Salzsäure) und erneuter Inkubation bei 37 °C (0,5 h) ein farbiges Addukt, dass colorimetrisch durch seine Absorption bei 560 nm quantifiziert werden konnte.

## Patentansprüche

1. Verfahren zur Herstellung von Aldehyden umfassend die Verfahrensschritte
A) in Kontakt Bringen eines vicinalen Diols mit einer Hydro-Lyase,
B) Trennung des in Verfahrensschritt A) gebildeten Aldehyds und der Hydro-Lyase,
C) mindestens eine Wiederholung der Verfahrensschritte A) und B) mit der Hydro-Lyase erhalten aus Verfahrensschritt B) und gegebenenfalls
D) Isolierung des gebildeten Aldehyds,
wobei das vicinale Diol in Verfahrensschritt A) Glycerin oder 1,2-Propandiol ist,
die Hydro-Lyase in Verfahrensschritt A) ausgewählt ist aus Glycerin-Dehydratasen, Diol-Dehydratasen und Propandiol-Dehydratasen
**dadurch gekennzeichnet,**
**dass** das Verfahren in Gegenwart einer mit dem Aldehyd eine Bindung ausbildenden Verbindung ausgewählt aus der Gruppe Hydrazide, Hydrazine, Hydrogen-Sulfite, Sulfite, Metabisulfite und Pyrosulfite
durchgeführt wird.

2. Verfahren gemäß Anspruch 1,
**dadurch gekennzeichnet,**
**dass** die in Verfahrensschritt A) eingesetzte Hydro-Lyase auf einem Träger immobilisiert ist.

3. Verfahren gemäß Anspruch 1 oder 2, **dadurch gekennzeichnet, dass** in Verfahrensschritt A) als Hydro-Lyase Hydro-Lyase-Aktivität aufweisende Zellen eingesetzt werden.

4. Verfahren gemäß Anspruch 3, **dadurch gekennzeichnet,**
**dass** die Zellen eine gegenüber ihrem Wildtyp verminderte Aktivität einer Oxidoreduktase aufweisen, welche den Aldehyd zu reduzieren vermag.

5. Verfahren gemäß mindestens einem der Ansprüche 1 bis 4,
**dadurch gekennzeichnet,**
**dass** das vicinale Diol in Verfahrensschritt A) in einem Konzentrationsbereich von 0,01 Gew.-% bis 50 Gew.-% bezogen auf die gesamte Reaktionsmischung eingesetzt wird.

6. Verfahren gemäß mindestens einem der Ansprüche 1 bis 5,
**dadurch gekennzeichnet,**
**dass** die Verfahrensschritte A), B) und C) kontinuierlich durchgeführt werden.

7. Verfahren gemäß mindestens einem der Ansprüche 1 bis 6,
**dadurch gekennzeichnet,**
**dass** als eine mit dem Aldehyd eine Bindung ausbildenden Verbindung Semicarbazid [CAS 57-56-7], Carbohydrazid [CAS 497-18-7] oder Natriumsulfit [CAS 7757-83-7] eingesetzt werden.

8. Verfahren gemäß mindestens einem der Ansprüche 1 bis 7,
**dadurch gekennzeichnet,**
**dass** in dem erfindungsgemäßen Verfahren das vicinale Diol und die funktionelle Gruppe der mit dem Aldehyd eine Bindung ausbildenden Verbindung in einem molaren Verhältnis von mindestens 1:1 eingesetzt werden.

9. Verfahren zur Herstellung von Oxidations- oder Reduktionsprodukten eines Aldehydes, umfassend die Verfahrensschritte:
I) Bereitstellung eines Aldehyds durch ein Verfahren gemäß einem der Ansprüche 1 bis 8,
II) chemische oder biokatalytische Umsetzung des Aldehyds durch Reduktion, Oxidation, Disproportionierung oder Dehydratisierung unter Erhalt von gegebenenfalls ungesättigen Reduktions- oder Oxidationszwischenprodukten und gegebenenfalls
III) die weitere chemische oder biokatalytische Umsetzung der im Verfahrensschritt II) erhaltenen Reduktions- oder Oxidationszwischenprodukte durch Reduktion, Oxidation oder Addition.

## Claims

1. Method for producing aldehydes, comprising the steps
A) bringing a vicinal diol into contact with a hydro-lyase,
B) separating the aldehyde formed in step A) and the hydro-lyase,
C) at least one repetition of steps A) and B) with the hydro-lyase obtained from step B) and optionally
D) isolating the aldehyde formed,
wherein the vicinal diol in step A) is glycerol or 1,2-propanediol,
the hydro-lyase in step A) is selected from glycerol dehydratases, diol dehydratases and propanediol dehydratases,
**characterized in that**
the method is carried out in the presence of a compound which forms a bond with the aldehyde, selected from the group consisting of hydrazides, hydrazines, hydrogensulfites, sulfites, metabisulfites and pyrosulfites.

2. Method according to Claim 1,
**characterized in that**
the hydro-lyase used in step A) is immobilized on a support.

3. Method according to Claim 1 or 2, **characterized in that**
in step A), cells having hydro-lyase activity are used as hydro-lyase.

4. Method according to Claim 3,
**characterized in that**
the cells have an activity, which is reduced compared to their wild type, of an oxidoreductase which is able to reduce the aldehyde.

5. Method according to at least one of Claims 1 to 4,
**characterized in that**
the vicinal diol in step A) is used in a concentration range from 0.01% by weight to 50% by weight, based on the total reaction mixture.

6. Method according to at least one of Claims 1 to 5,
**characterized in that**
steps A), B) and C) are carried out continuously.

7. Method according to at least one of Claims 1 to 6,
**characterized in that**
semicarbazide [CAS 57-56-7], carbohydrazide [CAS 497-18-7] or sodium sulfite [CAS 7757-83-7] are used as a compound which forms a bond with the aldehyde.

8. Method according to at least one of Claims 1 to 7,
**characterized in that**
in the method according to the invention, the vicinal diol and the functional group of the compound which forms a bond with the aldehyde are used in a molar ratio of at least 1:1.

9. Method for producing oxidation or reduction products of an aldehyde, comprising the steps:
I) provision of an aldehyde by a method according to one of Claims 1 to 8,
II) chemical or biocatalytic reaction of the aldehyde by reduction, oxidation, disproportionation or dehydration to give optionally unsaturated reduction or oxidation intermediates and optionally
III) the further chemical or biocatalytic reaction of the reduction or oxidation intermediates obtained in step II) by reduction, oxidation, or addition.

## Revendications

1. Procédé pour la production d'aldéhydes, comprenant les étapes de processus
A) mise en contact d'un diol vicinal avec une hydrolyase,
B) séparation de l'aldéhyde formé dans l'étape A) du processus et de l'hydro-lyase,
C) au moins une répétition des étapes A) et B) du processus avec l'hydro-lyase obtenue à partir de l'étape B) du processus et éventuellement
D) isolement de l'aldéhyde formé,
le diol vicinal dans l'étape A) du processus étant le glycérol ou le 1,2-propanediol,
l'hydro-lyase dans l'étape A) du processus étant choisie parmi les glycérol-déshydratases, les diol-déshydratases et les propanediol-déshydratases,
**caractérisé en ce**
**qu'**on effectue le procédé en présence d'un composé formant une liaison avec l'aldéhyde, choisi dans le groupe des hydrazides, hydrazines, hydrogénosulfites, sulfites, métabisulfites et pyrosulfites.

2. Procédé selon la revendication 1,
**caractérisé en ce que**
l'hydro-lyase utilisée dans l'étape A) du processus est immobilisée sur un support.

3. Procédé selon la revendication 1 ou 2,
**caractérisé en ce que**
dans l'étape A) du processus on utilise comme hydrolyase des cellules présentant une activité hydro-lyase.

4. Procédé selon la revendication 3,
**caractérisé en ce que**
les cellules présentent une activité d'une oxydoréductase, susceptible de réduire l'aldéhyde, qui est diminuée par rapport à leur type sauvage.

5. Procédé selon au moins l'une quelconque des revendications 1 à 4,
**caractérisé en ce que**
dans l'étape A) du processus on utilise le diol vicinal dans une plage de concentration de 0,01 % en poids à 50 % en poids, par rapport au mélange réactionnel total.

6. Procédé selon au moins l'une quelconque des revendications 1 à 5,
**caractérisé en ce que** les étapes A), B) et C) du processus sont effectuées en continu.

7. Procédé selon au moins l'une quelconque des revendications 1 à 6,
**caractérisé en ce**
**qu'**en tant qu'un composé formant une liaison avec l'aldéhyde on utilise le semicarbazide [CAS 57-56-7], le carbohydrazide [CAS 497-18-7] ou le sulfite de sodium [CAS 7757-83-7].

8. Procédé selon au moins l'une quelconque des revendications 1 à 7,
**caractérisé en ce que**
dans le procédé selon l'invention le diol vicinal et le groupe fonctionnel du composé formant une liaison avec l'aldéhyde sont utilisés en un rapport molaire d'au moins 1:1.

9. Procédé pour la préparation de produits d'oxydation ou de réduction d'un aldéhyde, comprenant les étapes de processus :
I) production d'un aldéhyde par un procédé selon l'une quelconque des revendications 1 à 8,
II) conversion chimique ou biocatalytique de l'aldéhyde par réduction, oxydation, dismutation ou déshydratation, avec obtention de produits intermédiaires de réduction ou d'oxydation éventuellement insaturés et éventuellement
III) la conversion chimique ou biocatalytique plus poussée, par réduction, oxydation ou addition, des produits intermédiaires de réduction ou d'oxydation obtenus dans l'étape II) du processus.
